# EUROPÄISCHE PATENTSCHRIFT

(11) **EP 0 444 504 B1**
(45) Veröffentlichungstag und Bekanntmachung des Hinweises auf die Patenterteilung: **05.04.1995**
(21) Anmeldenummer: 91102376.0
(22) Anmeldetag: 20.02.1991
(51) Int. Cl.: B01D 53/34, C01B 3/02, C07C 1/02, C07C 1/06

(54) **Verfahren zur Verwertung mindestens von Bestandteiles eines bei einer Verbrennung entstehenden Rauchgases**
Method of using at least one component of a flue gas
Méthode pour utiliser au moins un composant d'un gaz de fumée

(30) Priorität: 27.02.1990 DE 4006104; 18.08.1990 DE 4026201; 06.12.1990 DE 4038905
(43) Veröffentlichungstag der Anmeldung: 04.09.1991
(73) Patentinhaber: Fischer, Reinhard, D-52074 Aachen (DE)
(72) Erfinder: Fischer, Reinhard, Dr., W-5100 Aachen (DE); Menges, Georg, Prof.Dr.Ing., W-5100 Aachen (DE)
(74) Vertreter: Köckeritz, Günter

(56) Entgegenhaltungen:
- EP-A- 0 079 207
- EP-A- 0 148 684
- EP-A- 0 178 853
- WO-A-83/04270
- WO-A-89/03241
- DE-A- 3 525 479
- US-A- 3 660 023
- N.N. GREENWOOD et al.: "Chemie der Elemente", 1988, Seiten 367-368, VCH Verlagsgesellschaft, Weinheim, DE

## Beschreibung

In der WO-A-8903241 "Verfahren zur Beseitigung von Abfällen durch Verbrennen mit Sauerstoff" ist ein Verfahren beschrieben worden, bei dem Abfälle anstatt mit Verbrennungsluft mit hochprozentigem Sauerstoff verbrannt werden. Unter Abfällen sind dort u. a. Hausmüll, Sonderabfälle, Shreddergut und dgl. erwähnt worden. Die Abfälle werden in einem geeigneten Ofen mit Sauerstoff verbrannt, die Abgase in einem Abhitzekessel abgekühlt und dann in einer geeigneten Gasreinigungsanlage von Schadstoffen weitgehend befreit. Bei diesem Verbrennungsvorgang entsteht Rauchgas, und es werden vorhandene Kohlenwasserstoffe hauptsächlich zu Kohlendioxid und Wasser verbrannt und in die Umwelt entlassen. Da der Stickstoffbalast, der beim Verbrennen mit Luft vorhanden ist, fehlt, haben die Verbrennungsabgase nur geringe Stickstoff-Gehalte in Abhängigkeit vom Stickstoffgehalt in den zu verbrennenden Abfällen.
Kohlendioxid ist heutzutage ein sehr unerwünschtes Verbrennungsprodukt, da es zum sogenannten "Treibhauseffekt" beiträgt.
Aus der Literatur und der Praxis sind viele Verfahren bekannt, bei denen Kohlenoxide zu Kohlenwasserstoffen unterschiedlichster Zusammensetzung unter Verwendung geeigneter Katalysatoren hydriert werden.
Es seien hier z. B. Fischer-Tropsch Benzinsynthese

n CO + 2 n H₂ ⇄ H - (CH₂)n - H

oder die Methanol-Synthese

CO + 2 H₂ ⇄ CH₃ OH

erwähnt.
Auch Kohlenmonoxid und Wasserdampf lassen sich bei geeigneten Mischungsverhältnissen und bestimmten Katalysatoren in verschiedene Kohlenwasserstoffe umwandeln, wie Herr Kölbel und Frau Engelhardt nachgewiesen haben:

m CO + n H₂O ⇄ o CH₂ + p C₂H₅OH + q CO₂

Die Hydrierung von Kohlenmonoxid mit Wasserstoff ist ebenfalls möglich, wie z. B. die EP-A-0 079 207 "Process for the production of hydrocarbons and oxygenated derivatives thereof by the catalytic hydrogenation of carbondioxide" zeigt:

m CO₂ + n H₂ ⇄ o CH₄ + p CH₃OH + q CO

Während bisher für die Synthesen von Kohlenwasserstoffen eigens dafür hergestellte sogenannte Synthesegase verwendet wurden, aber auch schon Synthesen mit Gichtgasen und Wassergas durchgeführt wurden, ist erstmalig mit der DE-A 35 25 479 vorgeschlagen worden, aus der Verbrennung von Gasen entstandenes CO₂ bei hoher Temperatur mit Methan und Wasserdampf in ein hauptsächlich aus CO und H₂ bestehendes Gas umzuwandeln, das als Synthesegas für verschiedenste Synthese-Produkte geeignet ist.
Ausgehend von diesem Stand der Technik, liegt der Erfindung die Aufgabe zugrunde, ein Verfahren der eingangs beschriebenen Art vorzuschlagen, mit dem billige Rohstoffquellen für die Gewinnung von Kohlenwasserstoffen erschlossen werden können, wobei das Verfahren weitgehend emissionsfrei durchführbar sein soll, so daß insbesondere die Kohlendioxidemission vermieden wird.

Diese Aufgabe ist erfindungsgemäß dadurch gelöst, daß kohlenwasserstoffhaltige und/oder kohlenstoffhaltige Stoffe mit hochprozentigem Sauerstoff verbrannt und die bei der Verbrennung anfallenden Kohlendioxid und/oder Kohlenmonoxid sowie Wasserdampf enthaltenden Rauchgase zur Synthese von Kohlenwasserstoffen benutzt werden. Es kann somit in dem hier vorgeschlagenen Verfahren der primäre Brennstoff, nämlich kohlenstoff- bzw. kohlenwasserstoffhaltige Materialien, wie z. B. Müll, Shredderschutt, Sonderabfälle und dgl., mit sauerstoffhaltigen Gasen verbrannt werden, und es können die dabei anfallenden, im Rauchgas enthaltenen Kohlenoxide und ggf. Wasserdampf auf verschiedenste Art und Weise in Kohlenwasserstoffe verschiedener Zusammensetzung umgewandelt werden. Eine Reduktion des im Rauchgas enthaltenen CO₂ und ggf. auch des H₂O erfolgt durch Eindüsen von gasförmigen, flüssigen, granulierten oder zerkleinerten festen Abfall-Kohlenwasserstoffen, wie z. B. Altöl, Lösungsmitteln oder Kunststoffabfällen. Eine Kohlendioxidemission wird so vermieden.
Normalerweise enthalten die Rauchgase der Abfallverbrennung mit Sauerstoff - wie bereits oben erwähnt - hauptsächlich Kohlendioxid und Wasserdampf. Verbrennt man jedoch, wie nach der Erfindung weiter vorgeschlagen, mit einem Sauerstoff-Unterschuß, lassen sich bestimmte CO-Gehalte im Rauchgas einstellen.

Eine andere, nach der Erfindung noch vorgeschlagene Möglichkeit der Erzeugung von CO im Abgas besteht darin, erst eine vollständige Verbrennung der Abfälle mit Sauerstoff zu Kohlendioxid und Wasserdampf durchzuführen und dann in das heiße Abgas durch Eindüsen flüssiger Abfall-Kohlenwasserstoffe das Kohlendioxid zu Kohlenmonoxid zu reduzieren. Es kann weiter zusätzlich Wasser in das heiße Gas eingedüst und in Dampf umgewandelt werden. Hierbei ist ein besonders günstiges Verhältnis von H₂O : CO ein Verhältnis von 1,0 - 1,5:3. Leitet man dieses Gasgemisch bei Normaldruck oder erhöhtem Druck bei Temperaturen von 220 - 300 °C über geeignete Katalysatoren, so entstehen Kohlenwasserstoffe unterschiedlichster Zusammensetzung. Mit Hilfe von Sauerstoff können sie auch in Kohlenwasserstoffderivate umgewandelt werden.
Durch Eindüsen von flüssigen Abfall-Kohlenwasserstoffen und Wasser in das heiße Rauchgas kann ein Synthesegas erzeugt werden, das hauptsächlich aus CO und H₂ besteht:

C₃H₈ + 3 H₂O ⇄ 3 CO + 7 H₂

C₃H₈ + 3 CO₂ ⇄ 6 CO + 4 H₂

Je größer das Verhältnis Wasserstoff zu Kohlenstoff in den Abfall-Kohlenwasserstoffen ist, umso günstiger ist das Verhältnis des aus der Müllverbrennung stammenden CO₂ zu dem benötigten Abfall-Kohlenwasserstoff.
Man kann jedoch auch die CO₂-haltigen Rauchgase unmittelbar mit H₂ hydrieren. Der hierfür benötigte Wasserstoff kann durch die Zerlegung von Wasser gewonnen werden, wobei der dabei anfallende Sauerstoff sofort zur Verbrennung der kohlenstoff- und/oder kohlenwasserstoffhaltigen Stoffe verwendet werden kann. Die Energie zur Wasserzerlegung gewinnt man vorteilhafterweise aus der Verbrennung der kohlenstoff- und/oder kohlenwasserstoffhaltigen Stoffe selbst. Vorteilhafterweise können als Reduktionsmittel neben gasförmigen Abfall-Kohlenwasserstoffen insbesondere auch granulierte oder pulverisierte Abfall-Kohlenwasserstoffe, wie Kunststoffabfälle in beliebiger Kombination, verwendet werden. Dies ist möglich, weil diese Kunststoffabfälle bei z. B. 1000 °C in 0,5 Sek. rückstandsfrei vergasen.
Auch weitere Verwertungen oder Anwendungen der beschriebenen Rauchgase sind denkbar. So wird z. B. nach der Erfindung vorgeschlagen, die bei der Verbrennung von kohlenwasserstoffhaltigen und/oder kohlenstoffhaltigen Stoffen entstehenden Rauchgase in bestehenden Anlagen zur Erzeugung von Synthesegas, Brenngas, Reduktionsgas oder anderen verwertbaren Gasen mindestens zusätzlich zu den dort bisher verwendeten Stoffen einzubringen. Diese Maßnahme kann vor oder nach der Reduktion erfolgen, und es gelingt hierdurch, zusätzliche Kohlendioxidemissionen und sonstige Schadstoffemissionen zu vermeiden, und es können mit dieser Maßnahme die dort bisher verwendeten Stoffe teilweise oder auch vollständig ersetzt werden.

Das erfindungsgemäße Verfahren wird wie folgt beschrieben:

### Variante 1

Die Verbrennung der genannten Stoffe mit Sauerstoff erfolgt in bekannter Weise. In die heißen Rauchgase werden kohlenwasserstoffhaltige, flüssige, gasförmige oder granulierte Abfallstoffe, wie z. B. PCB-haltiges Abfallöl oder dgl., oder feste kohlenwasserstoffhaltige Abfallstoffe, wie z. B. Polyolefine, vor dem Abhitzekessel eingebracht. Dadurch erfolgt eine Umwandlung

CO₂ + H₂O + KW ⇄ CO + H₂

Die Rauchgase verlassen den Verbrennungsofen mit einer Temperatur über 1 200 °C, passieren einen Abhitzekessel und werden nach Abkühlung auf ca. 300 °C in einer Gasreinigungsanlage, z. B. einem Hochtemperatur-Gewebefilter aus Teflon, entstaubt. Anschließend werden sie in einer üblichen und bekannten Synthese-Vorrichtung, die mit entsprechenden Katalysatoren ausgestattet ist, in die gewünschten Kohlenwasserstoffe oder sauerstoffhaltigen Kohlenwasserstoff-Derivate umgewandelt.

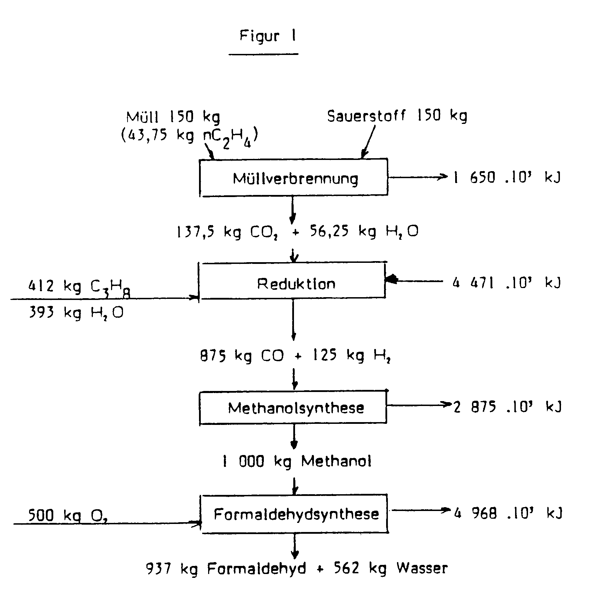

In der Figur 1 ist ein Beispiel für die Variante 1 dargestellt.

### Variante 2

Diese Variante ist eine Abwandlung der Variante 1 insoweit, als nur CO₂ durch Eindüsen von flüssigen Abfall-Kohlenwasserstoffen zu CO reduziert wird. Gleichzeitig wird in die heißen Gase vor oder nach dem Abhitzekessel soviel Wasser eingespritzt, daß das bekannte optimale Verhältnis CO zu H₂O je nach gewünschter Kohlenwasserstoffverbindung eingestellt wird.

## Patentansprüche

1. Verfahren zur Verwertung der bei der Verbrennung von kohlenwasserstoffhaltigen und/oder kohlenstoffhaltigen Stoffen mit hochprozentigem Sauerstoff entstehenden Rauchgase, wobei in das Rauchgas gasförmige, flüssige oder granulierte oder zerkleinerte feste Abfall-Kohlenwasserstoffe zur Reduktion von im Rauchgas enthaltenen Kohlendioxid und Wasserdampf eingedüst werden und die dabei entstehenden Gase einer Umwandlung in Kohlenwasserstoffe zugeführt werden.

2. Verfahren nach Anspruch 1 dadurch gekennzeichnet, daß
es sich bei den zur Verbrennung gelangenden Stoffen um Abfälle, insbesondere Müll, Sondermüll oder Shreddergut, handelt.

3. Verfahren nach den Ansprüchen 1 und 2
dadurch gekennzeichnet, daß
es sich bei den Abfall-Kohlenwasserstoffen um Altöl, Lösungsmittel oder Kunststoffabfälle handelt.

4. Verfahren nach den Ansprüchen 1 bis 3
dadurch gekennzeichnet, daß
die Verbrennung mit hochprozentigem Sauerstoff mit einem Sauerstoffunterschuß durchgeführt wird.

5. Verfahren nach den Ansprüchen 1 bis 4
dadurch gekennzeichnet, daß
die entstehenden Gase vor einer Umwandlung in Kohlenwasserstoffe abgekühlt und gereinigt werden.

6. Verfahren nach den Ansprüchen 1 bis 5
dadurch gekennzeichnet, daß
in das heiße Rauchgas Abfall-Kohlenwasserstoffe und Wasser eingedüst werden, woraufhin dieses Gasgemisch vor der Weiterverwendung abgekühlt und gereinigt wird.

7. Verfahren nach Anspruch 6 dadurch gekennzeichnet, daß
Abfall-Kohlenwasserstoffe und Wasser in einer Menge eingedüst werden, daß sich ein H₂O : CO-Verhältnis von 1,0 - 1,5 : 3 einstellt.

8. Verfahren nach einem der Ansprüche 1 bis 7
dadurch gekennzeichnet, daß
die behandelten Rauchgase in bestehende Anlagen zur Erzeugung von Synthesegas, Brenngas, Reduktionsgas oder anderen verwertbaren Gasen mindestens zusätzlich zu den dort bisher verwendeten Stoffen eingebracht werden.

9. Verfahren nach den Ansprüchen 1 bis 8
dadurch gekennzeichnet, daß
der zur Hydrierung benötigte Wasserstoff durch die Zerlegung von Wasser gewonnen und der dabei anfallende Sauerstoff für die Verbrennung der kohlenstoff- und/oder kohlenwasserstoffhaltigen Stoffe verwendet wird.

## Claims

1. Method of utilising the flue gases which result from burning hydrocarbon-containing and/or carbon-containing substances with high-percentage oxygen, wherein gaseous, liquid or granulated or comminuted solid waste hydrocarbons are sprayed into the flue gas to reduce carbon dioxide and water vapour contained in the flue gas, and the gases resulting therefrom are converted into hydrocarbons.

2. Method according to claim 1, characterised in that the substances being burned are waste materials, more especially refuse, special refuse or shredded material.

3. Method according to claims 1 and 2, characterised in that the waste hydrocarbons are used oil, solvents or waste plastics materials.

4. Method according to claims 1 to 3, characterised in that the combustion with high-percentage oxygen is effected with a deficiency of oxygen.

5. Method according to claims 1 to 4, characterised in that the resultant gases are cooled and purified prior to being converted into hydrocarbons.

6. Method according to claims 1 to 5, characterised in that waste hydrocarbons and water are sprayed into the hot flue gas, whereupon this gas mixture is cooled and purified prior to re-use.

7. Method according to claim 6, characterised in that waste hydrocarbons and water are sprayed-in in such a quantity that an H₂O : CO ratio of 1.0 - 1.5 : 3 is set.

8. Method according to one of claims 1 to 7, characterised in that the treated flue gases are introduced into existing systems for producing synthetic gas, combustion gas, reduction gas or other utilisable gases, at least in addition to the substances used there hitherto.

9. Method according to claims 1 to 8, characterised in that the hydrogen, required for hydrogenation, is obtained by the decomposition of water, and the oxygen resulting therefrom is used for the combustion of the carbon- and/or hydrocarbon-containing substances.

## Revendications

1. Procédé de valorisation des gaz de fumée formés lors de la combustion de substances contenant des hydrocarbures et/ou contenant du carbone, avec de l'oxygène à haute teneur, suivant lequel, dans le gaz de fumée, sont injectés des déchets d'hydrocarbures gazeux, liquides ou solides granulés ou pulvérisés, en vue de la réduction du dioxyde de carbone et de la vapeur d'eau contenus dans le gaz de fumée, et les gaz ainsi formés sont soumis à une conversion en hydrocarbures.

2. Procédé selon la revendication 1, caractérisé par le fait que les substances parvenant à la combustion, sont des déchets, en particulier des ordures, des ordures spéciales ou des matériaux déchiquetés.

3. Procédé selon les revendications 1 et 2, caractérisé par le fait que les déchets d'hydrocarbures sont une huile usée, des solvants ou des déchets de matières plastiques.

4. Procédé selon les revendications 1 à 3, caractérisé par le fait que la combustion avec de l'oxygène à haute teneur est conduite avec un déficit en oxygène.

5. Procédé selon les revendications 1 à 4, caractérisé par le fait que les gaz qui se forment sont refroidis et épurés avant une conversion en hydrocarbures.

6. Procède selon les revendications 1 à 5, caractérisé par le fait que, dans le gaz de fumée chaud, sont injectés des déchets d'hydrocarbures et de l'eau, sur quoi ce mélange de gaz est refroidi et épuré avant l'utilisation ultérieure.

7. Procédé selon la revendication 6, caractérisé par le fait que les déchets d'hydrocarbures et l'eau sont injectés dans une quantité telle que l'on parvienne à un rapport H₂O:CO de 1,0-1,5:3.

8. Procédé selon l'une des revendications 1 à 7, caractérisé par le fait que les gaz de fumée traités sont chargés dans des installations existantes pour l'obtention de gaz de synthèse, de gaz combustible, de gaz de réduction ou d'autres gaz intéressants au moins additionnellement aux substances qui y étaient utilisées jusqu'ici.

9. Procédé selon les revendications 1 à 8, caractérisé par le fait que l'hydrogène nécessaire à l'hydrogénation est obtenu par la décomposition de l'eau, et l'oxygène qui se forme alors est utilisé pour la combustion des substances contenant du carbone et/ou contenant des hydrocarbures.
